# EUROPÄISCHE PATENTSCHRIFT

(11) **EP 3 694 388 B1**
(45) Veröffentlichungstag und Bekanntmachung des Hinweises auf die Patenterteilung: **19.04.2023**
(21) Anmeldenummer: 18782440.4
(22) Anmeldetag: 04.10.2018
(51) Int. Cl.: A61B 1/00

(54) **OPTISCHES SYSTEM FÜR EIN STEREO-VIDEOENDOSKOP**
OPTICAL SYSTEM FOR A STEREO-VIDEO ENDOSCOPE
SYSTÈME OPTIQUE POUR UNE CAMÉRA STÉRÉOSCOPIQUE

(30) Priorität: 13.10.2017 DE 102017123896
(43) Veröffentlichungstag der Anmeldung: 19.08.2020
(73) Patentinhaber: Olympus Winter & Ibe GmbH, 22045 Hamburg (DE)
(72) Erfinder: WIETERS, Martin, 22885 Barsbüttel (DE); SCHOUWINK, Peter, 22926 Ahrensburg (DE); ZHAO, Jianxin, 22399 Hamburg (DE); THÜMEN, Alrun, 22043 Hamburg (DE)
(74) Vertreter: Seemann & Partner Patentanwälte mbB
(86) Internationale Anmeldenummer: PCT/EP2018/077037
(87) Internationale Veröffentlichungsnummer: WO 2019/072685

(56) Entgegenhaltungen:
- EP-A1- 2 806 301
- EP-A2- 3 037 864
- JP-A- 2001 145 640
- US-A1- 2005 001 899
- US-A1- 2009 040 606
- US-A1- 2009 160 935
- US-A1- 2015 168 710

## Beschreibung

Die Erfindung betrifft ein optisches System für ein Stereo-Videoendoskop sowie ein Stereo-Videoendoskop.

In der Medizin werden Stereo-Videoendoskope eingesetzt, um einem behandelnden Arzt eine räumliche Darstellung des Körperinneren eines Patienten zu verschaffen. Dazu werden in ein optisches System des Endoskops einfallende Lichtbündel in zwei parallel verlaufenden und ausgebildeten Linsensystemkanälen geführt, welche die Lichtbündel auf zwei getrennte Bildsensoren abbilden. Auf diese Weise werden Bilder des betrachteten Bereichs unter leicht unterschiedlichen Blickwinkeln erfasst. Werden diese Bilder so betrachtet, dass jedes Auge jeweils das Bild oder die Bilder eines Linsensystemkanals wahrnimmt, beispielsweise mit Hilfe einer Shutterbrille, so entsteht ein räumlicher Eindruck des betrachteten Bereichs. Dies wird als Stereoskopie bezeichnet. Ein solches Stereo-Videoendoskop ist beispielsweise in DE 10 2013 215 422 A1 offenbart.

Für die Stärke des räumlichen Eindrucks ist der Abstand der optischen Achsen der Linsensystemkanäle entscheidend. Ist dieser Abstand zu klein, so tritt nur ein schwacher räumlicher Eindruck auf. Bei der Konstruktion eines Stereo-Videoendoskops ist daher auf den richtigen Abstand der Linsensystemkanäle zu achten. Gleichzeitig ist bei der Konstruktion von Endoskopen darauf zu achten, dass der Außendurchmesser der Endoskope möglichst klein ist, um die mit einer endoskopischen Untersuchung verbundene Belastung für den Patienten zu minimieren. Eine Schwierigkeit ergibt sich dadurch, dass die in den Linsensystemkanälen angeordneten optischen Elemente, beispielsweise Linsen, eine möglichst große Querschnittsfläche aufweisen sollen, damit eine hohe Bildqualität erzielt wird. Sind diese optischen Elemente zu groß, so können sie nicht mehr nebeneinander in dem Hüllrohr des Endoskops angeordnet werden. Zudem muss weiterhin darauf geachtet werden, dass auch bei großen optischen Elementen der richtige Abstand der optischen Achsen eingehalten wird. Bei Stereo-Videoendoskopen muss also ein Kompromiss zwischen diesen Anforderungen gefunden werden.

US 2009/0160935 A1 zeigt die Merkmale des Oberbegriffs des Anspruchs 1 und offenbart eine Bildteilungsvorrichtung für ein Videoendoskop mit einer optischen Komponente zur Bildteilung, mit der auf einem Videosensor ein einziges Kompositbild aus zwei Bildern eines beobachteten Zielobjekts gebildet wird, das aus zwei verschiedenen Richtungen betrachtet wird. Die optische Komponente umfasst zwei Abschnitte identischer konvergenter Linsen, die in ein opakes zentrales Element eingebettet sind, welches einen Abstand zwischen den beiden Linsenabschnitten wahrt. Jeder der beiden Linsenabschnitte ist wenigstens gleich einem Halbmond, so dass es von der optischen Achse der Linse durchquert wird.

Aus EP 2 806 301 A1 ist eine optische Anordnung für ein Stereoendoskop bekannt, bei dem Linsen zur Verringerung des Abstands der parallelen optischen Achsen der Linsensysteme jeweils auf den einander zuweisenden Seiten mit einem geraden Schnitt versehen sind.

Die Aufgabe der Erfindung besteht darin, ein optisches System für ein Stereo-Videoendoskop sowie ein Stereo-Videoendoskop bereitzustellen, mit dem eine Verbesserung der Bildqualität, des stereoskopischen Effekts und/oder eine Vergrößerung des Blickfelds erreicht wird, ohne den Außendurchmesser des Endoskopschafts zu vergrößern.

Diese Aufgabe wird gelöst durch ein optisches System für ein Stereo-Videoendoskop mit einem ersten Linsensystemkanal und einem zweiten Linsensystemkanal für eine stereoskopische Darstellung eines außerhalb des Stereo-Videoendoskops liegenden Bereichs, wobei die Linsensystemkanäle parallel zueinander angeordnet sind und jeweils ein oder mehrere optische Elemente in jeweils gleicher optischer Konfiguration umfassen, die jeweils in gleicher Position entlang einer ersten optischen Achse des ersten Linsensystemkanals bzw. einer zweiten optischen Achse des zweiten Linsensystemkanals nebeneinander angeordnet sind, wobei der erste Linsensystemkanal wenigstens ein erstes optisches Element und der zweite Linsensystemkanal wenigstens ein dem ersten optischen Element benachbartes zweites optisches Element umfasst, wobei eine erste optische Achse des ersten optischen Elements mit der ersten optischen Achse des ersten Linsensystemkanals zusammenfällt und eine zweite optische Achse des zweiten optischen Elements mit der zweiten optischen Achse des zweiten Linsensystemkanals zusammenfällt, wobei die Querschnittsfläche des ersten optischen Elements in einen ersten Umfangskreis und die Querschnittsfläche des zweiten optischen Elements in einen zweiten Umfangskreis eingeschrieben sind, deren Zentren jeweils mit der ersten optischen Achse des ersten optischen Elements bzw. der zweiten optischen Achse des zweiten optischen Elements zusammenfallen und die einen maximalen Radius des ersten optischen Elements bzw. des zweiten optischen Elements bestimmen, wobei der erste Umfangskreis und der zweite Umfangskreis einander überlappen, wobei Umfangsformen des ersten optischen Elements und des zweiten optischen Elements von den sie umschreibenden ersten und zweiten Umfangskreisen derart abweichen, dass sich das erste optische Element und das zweite optische Element nicht berühren, wobei der erste Linsensystemkanal einen ersten Bildsensor und ein erstes Umlenkelement und der zweite Linsensystemkanal einen zweiten Bildsensor und ein zweites Umlenkelement umfassen, wobei der erste Bildsensor oberhalb und der zweite Bildsensor unterhalb einer von der ersten optischen Achse des ersten optischen Elements und der zweiten optischen Achse des zweiten optischen Elements aufgespannten Schnittfläche angeordnet und beide Bildsensoren planparallel zur Schnittfläche ausgerichtet sind, wobei das erste Umlenkelement in den ersten Linsensystemkanal einfallende Lichtbündel in Richtung des ersten Bildsensors und das zweite Umlenkelement in den zweiten Linsensystemkanal einfallende Lichtbündel in Richtung des zweiten Bildsensors ablenkt.

Im Rahmen der vorliegenden Erfindung bedeutet das Merkmal, dass die Einschnittsfläche eines optischen Elements in einen Umfangskreis eingeschrieben ist, dass es nicht über den Umfangskreis hinausragt, diesen aber zumindest teilweise oder punktuell berührt.

Bei Stereo-Videoendoskopen nach dem Stand der Technik entsprechen die umschreibenden Umfangskreise dem Umfang der kreisförmigen optischen Elemente, also beispielsweise dem Umfang der Linsen. Erfindungsgemäß weicht die Querschnittsfläche der optischen Elemente wenigstens abschnittsweise von diesen Umfangskreisen ab. Im Vergleich zu den Umfangskreisen ist der Umfang der optischen Elemente wenigstens in einem dem jeweils anderen optischen Element zugewandten Abschnitt des Umfangs kleiner. Die optischen Elemente weisen somit im Vergleich zu kreisrunden optischen Elementen wenigstens eine Aussparung auf. Dadurch ist es möglich, das erste optische Element und das zweite optische Element so nahe beieinander anzuordnen, dass sich die Umfangskreise überlappen. Die Größe der optischen Elemente ist also nicht mehr vom Abstand der optischen Achsen der beiden Linsensystemkanäle beschränkt.

Vorteilhaft kann dadurch der Abstand zwischen den optischen Achsen der Linsensystemkanäle und damit die Stärke des räumlichen Eindrucks angepasst werden. Insbesondere kann dieser Abstand reduziert werden, um einer Wahrnehmung als zwei getrennte Bilder entgegenzuwirken.

Weiterhin vorteilhaft können in einem Endoskop mit vorgegebenem Außendurchmesser und vorgegebenem Abstand zwischen den optischen Achsen der Linsensystemkanäle optische Elemente mit einem größeren Durchmesser verwendet werden. Durch die Aussparungen der optischen Elemente können diese näher zusammengeschoben werden. Dadurch wird zwischen den optischen Elementen und dem Hüllrohr des Endoskops Raum frei, der durch eine Vergrö-ßerung des Durchmessers der optischen Elemente nutzbar ist. Insgesamt wird dadurch eine höhere Bildqualität als mit optischen Elementen nach dem Stand der Technik erzielt, da der größere mögliche Durchmesser der optischen Elemente den Verlust an Fläche durch die Aussparungen überkompensiert.

Vorzugsweise weist die Querschnittsfläche des ersten optischen Elements und die Querschnittsfläche des zweiten optischen Elements jeweils die Form eines Kreissegments auf, wobei die Querschnittsfläche des ersten optischen Elements das Zentrum des ersten Umfangskreises und die Querschnittsfläche des zweiten optischen Elements das Zentrum des zweiten Umfangskreises umfassen, wobei eine die Querschnittsfläche des ersten optischen Elements begrenzende erste Kreissehne und eine die Querschnittsfläche des zweiten optischen Elements begrenzende zweite Kreissehne jeweils senkrecht zu einer Verbindungslinie zwischen den Zentren der Umfangskreise angeordnet sind.

Unter einem Kreissegment wird im Kontext der vorliegenden Beschreibung ein Segment eines Kreises verstanden, das von einem Kreisbogen und einer Kreissehne begrenzt ist. Ein Kreissektor, der von einem Kreisbogen und zwei Kreisradien begrenzt wird, also die Form eines Kuchenstücks hat, ist kein Kreissegment.

Die Form eines Kreissegments stellt eine fertigungstechnisch leicht zu realisierende Ausführungsform eines erfindungsgemäßen optischen Elements dar. Dabei wird beim ersten optischen Element und beim zweiten optischen Element ausgehend von kreisrunden optischen Elementen ein Teil der Querschnittsfläche entlang einer Kreissehne abgetrennt. Der verbleibende Teil ist dabei größer als der abgetrennte Teil, er umfasst also das Zentrum des Umfangkreises, die optische Achse. Die resultierende Form entspricht in etwa dem Buchstaben "D", weshalb man in diesem Fall von einem "D-Cut" spricht. In den Linsensystemkanälen werden die beiden optischen Elemente so ausgerichtet, dass die durch die Kreissehnen definierten Kanten der beiden optischen Elemente einander zugewandt sind.

Vorzugsweise ist das erste optische Element zum zweiten optischen Element spiegelsymmetrisch in Bezug auf eine Mittellinie, die mittig zwischen dem Zentrum der Umfangskreise angeordnet und senkrecht auf einer Verbindungslinie zwischen den Zentren der Umfangskreise steht.

Das erste optische Element im ersten Linsensystemkanal und das zweite optische Element im zweiten Linsensystemkanal sind gemäß dieser Ausführungsform also nicht nur nebeneinander angeordnet, sondern in Bezug auf die Mittellinie auch symmetrisch. Die Querschnittsflächen der optischen Elemente haben somit dieselbe Form und eine um die Mittellinie gespiegelte Ausrichtung.

Vorzugsweise sind im ersten Linsensystemkanal mehrere optische Elemente und im zweiten Linsensystemkanal dazu spiegelbildlich angeordnete optische Elemente umfasst. Durch die spiegelbildliche paarweise Anordnung aller optischen Elemente in den Linsensystemkanälen sind die optischen Eigenschaften der Linsensystemkanäle spiegelbildlich gleich. Vorzugsweise weisen nur die paarweise angeordneten optischen Elemente eine Aussparung auf, deren Umfangskreise sich überlappen.

Um eine hohe Bildqualität des stereoskopischen Bilds zu realisieren werden lichtstarke Optiken benötigt, welche die einfallenden Lichtbündel auf Bildsensoren mit entsprechend hoher Auflösung abbilden. Dies erfordert optische Elemente mit einem entsprechend gro-ßen Durchmesser. Bei einem Stereo-Videoendoskop mit einem Außendurchmesser von 10 mm liegt der Mindestdurchmesser der optischen Elemente unter Verwendung von modernen Bildsensoren mit hoher Auflösung bei etwa 3,4 mm. Gleichzeitig ist es zur Realisierung eines stereoskopischen Effekts notwendig, dass der Abstand zwischen den optischen Achsen der Linsensystemkanäle zwischen 2,5 mm und 3 mm liegt. Mit optischen Elementen, die eine vollständig kreisförmige Querschnittsfläche aufweisen, ist dies nicht zu realisieren, da sich in diesem Fall die Querschnittsflächen überlappen würden. Vorzugsweise weisen daher die die Querschnittsflächen umschreibenden Umfangskreise der optischen Elemente einen Durchmesser von wenigstens 3,4 mm auf und ein Abstand zwischen den Zentren der Umfangskreise beträgt 2,5 mm bis 3 mm.

Mittels der erfindungsgemäßen optischen Elemente, die beispielsweise einen D-Cut aufweisen, können beide Bedingungen erfüllt werden. Der Lichtverlust, der durch die Aussparung der optischen Elemente hervorgerufen wird, ist dabei so klein, dass er keinen wesentlichen Einfluss auf die Bildqualität hat. Vorteilhaft ist es somit möglich, lichtstarke Optiken und hochauflösende Bildsensoren auch in Stereo-Videoendoskopen mit einem Außendurchmesser von 10 mm zu verwenden und somit eine hohe Bildqualität und ein stereoskopisches Bild zu erzielen.

Erfindungsgemäß umfasst der erste Linsensystemkanal einen ersten Bildsensor und ein erstes Umlenkprisma und der zweite Linsensystemkanal einen zweiten Bildsensor und ein zweites Umlenkprisma, wobei der erste Bildsensor oberhalb und der zweite Bildsensor unterhalb einer von der ersten optischen Achse des ersten optischen Elements und der zweiten optischen Achse des zweiten optischen Elements aufgespannten Schnittfläche angeordnet und beide Bildsensoren planparallel zur Schnittfläche ausgerichtet sind, wobei das erste Umlenkelement in den ersten Linsensystemkanal einfallende Lichtbündel in Richtung des ersten Bildsensors und das zweite Umlenkelement in den zweiten Linsensystemkanal einfallende Lichtbündel in Richtung des zweiten Bildsensors ablenkt.

Dies erlaubt eine raumsparende Gestaltung des optischen Systems. Dazu ist es vorteilhaft, die Bildsensoren platzsparend anzuordnen. Üblicherweise sind die Bildsensoren so angeordnet, dass eine Normale auf der Fläche der Bildsensoren in Richtung der optischen Achsen der Linsensystemkanäle zeigt. Durch die Verwendung von Umlenkelementen, bei denen es sich beispielsweise um Prismen oder Spiegel handelt, können die Bildsensoren ober- bzw. unterhalb und planparallel zur Schnittfläche angeordnet werden. Auf diese Weise wird der von den Bildsensoren beanspruchte Platz reduziert.

Die Bildsensoren umfassen jeweils eine lichtempfindliche Fläche und einen lichtunempfindlichen Rand, wobei sich vorzugsweise die Bildsensoren, insbesondere die lichtunempfindlichen Ränder der Bildsensoren, in einer Projektion auf die Schnittfläche überlappen.

Damit wird erreicht, dass die in Stereo-Videoendoskopen verwendeten Bildsensoren, beispielsweise CCD-Sensoren, herstellungsbedingt einen lichtunempfindlichen Rand aufweisen, so dass, wenn die beiden Bildsensoren in derselben Ebene angeordnet sind, die Ränder verhindern, dass die Bildsensoren so zusammengeschoben werden, dass die lichtempfindlichen Flächen aneinander anliegen. Damit wird nutzbarer Platz verschenkt. Da die erfindungsgemäßen Bildsensoren jedoch nicht in der gleichen Ebene liegen, können sie so angeordnet werden, dass sich die lichtunempfindlichen Ränder in einer Projektion auf die Schnittfläche überlappen. Die Bildsensoren können auch noch weiter zusammengeschoben werden, so dass sich die lichtempfindlichen Flächen in der Projektion teilweise oder vollständig überlappen. Dadurch wird das optische System raumsparender gestaltet. Dies ermöglicht eine Verwendung von Bildsensoren mit größeren lichtempfindlichen Flächen, wodurch die Bildqualität erhöht wird. Zudem kann der Abstand zwischen den optischen Achsen der Linsensystemkanäle reduziert werden, wenn zuvor die Größe der Bildsensoren den minimalen Abstand der optischen Achsen vorgegeben hat. Bei großen Bildsensoren ist es durch die erfindungsgemäße Anordnung der Bildsensoren überhaupt erst möglich, einen stereoskopischen Effekt zu erzielen, da bei einer Anordnung der Bildsensoren in einer Ebene der Abstand zwischen den optischen Achsen zu groß wäre.

Vorzugsweise sind die Bildsensoren jeweils auf einem Träger angeordnet, wobei die Träger in einem in Bezug auf ein Hüllrohr des optischen Systems nahen Bereich dünner als in einem in Bezug auf das Hüllrohr entfernten Bereich sind. Die Träger sind auf der Seite der Bildsensoren angeordnet, die der lichtempfindlichen Seite der Bildsensoren entgegengesetzt ist. Der Bereich der Träger, der am dichtesten am Hüllrohr anliegt, begrenzt den minimalen Umfang des Hüllrohrs. Indem dieser Bereich dünner gestaltet wird, kann ein Hüllrohr mit einem kleineren Durchmesser oder Bildsensoren mit größeren Trägern verwendet werden. Da Bildsensoren mit größeren Trägern auch größere lichtempfindliche Flächen aufweisen, kann auf diese Weise die Bildqualität des optischen Systems verbessert werden.

Vorteilhafterweise fällt Licht durch ein Eintrittsfenster in das optische System ein, wobei das Eintrittsfenster eine Eintrittsfläche und eine Austrittsfläche umfasst und die Eintrittsfläche und die Austrittsfläche endliche und zueinander identische Krümmungsradien aufweisen. Die Fläche des Eintrittsfensters gibt vor, wieviel Licht in das optische System einfallen kann. Es ist daher eine Voraussetzung für eine hohe Bildqualität des optischen Systems, dass diese Fläche möglichst groß ist. Der Durchmesser des Eintrittsfensters wird jedoch durch den Endoskopdurchmesser begrenzt. Um die Fläche des Eintrittsfensters zu erhöhen, ohne den Durchmesser des Eintrittsfensters zu erhöhen, weist die Eintrittsfläche vorteilhafterweise eine Krümmung auf. Damit durch die Krümmung der Eintrittsfläche kein unerwünschter Linseneffekt des Eintrittsfensters auftritt, weist die Austrittsfläche eine Krümmung mit identischem Krümmungsradius auf. Auf diese Weise wird eine lichtstärkere Optik des optischen Systems realisiert und das Blickfeld vergrößert, ohne die Bildqualität durch einen unerwünschten Linseneffekt des Eintrittsfensters zu reduzieren.

In einer weiteren Ausführungsform des optischen Systems sind ein optisches Element im ersten Linsensystemkanal und ein gleichartiges optisches Element im zweiten Linsensystemkanal versetzt zueinander angeordnet. Auf diese Weise können unterschiedliche Weglängen der Lichtstrahlen, die bei einem seitwärts blickenden Stereo-Videoendoskop auftreten, bei Eintritt in die Linsensystemkanäle ausgeglichen werden.

Die Aufgabe wird außerdem gelöst durch ein Stereo-Videoendoskop umfassend ein optisches System in einer der zuvor beschriebenen Ausführungsformen. Das Stereo-Videoendoskop hat die gleichen oder ähnlichen Vorteile, Eigenschaften oder Merkmale, wie das zuvor beschriebene optische System.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: einen schematischen Längsschnitt durch ein optisches System eines Stereo-Videoendoskops,
- Fig. 2a: einen schematischen Querschnitt durch ein optisches System eines Stereo-Videoendoskops nach dem Stand der Technik,
- Fig. 2b: einen schematischen Querschnitt durch ein optisches System eines Stereo-Videoendoskops mit vergrößertem Hüllrohrdurchmesser,
- Fig. 2c: einen schematischen Querschnitt durch ein optisches System eines Stereo-Videoendoskops mit überlappenden optischen Elementen,
- Fig. 2d: einen schematischen Querschnitt durch ein optisches System eines Stereo-Videoendoskops mit optischen Elementen mit einem D-Cut,
- Fig. 3a: einen schematischen Querschnitt eines optischen Systems eines Stereo-Videoendoskops auf Höhe der Bildsensoren,
- Fig. 3b: eine schematische Darstellung eines Bildsensors,
- Fig. 4: eine schematische Draufsicht auf ein optisches System eines Stereo-Videoendoskops auf Höhe der Bildsensoren,
- Fig. 5: ein schematischer Querschnitt durch ein optisches System eines Stereo-Videoendoskops mit Bildsensoren mit im hüllrohrnahen Bereich dünneren Trägern,
- Fig. 6a: eine schematische Darstellung eines optischen Systems eines Stereo-Videoendoskops nach dem Stand der Technik,
- Fig. 6b: eine schematische Darstellung eines optischen Systems eines Stereo-Videoendoskops mit gekrümmtem Eintrittsfenster,
- Fig. 6c: eine schematische Darstellung eines optischen Systems eines Stereo-Videoendoskops mit gegeneinander verschobenen optischen Elementen.

In den Zeichnungen sind jeweils gleiche oder gleichartige Elemente und/oder Teile mit denselben Bezugsziffern versehen, so dass von einer erneuten Vorstellung jeweils abgesehen wird.

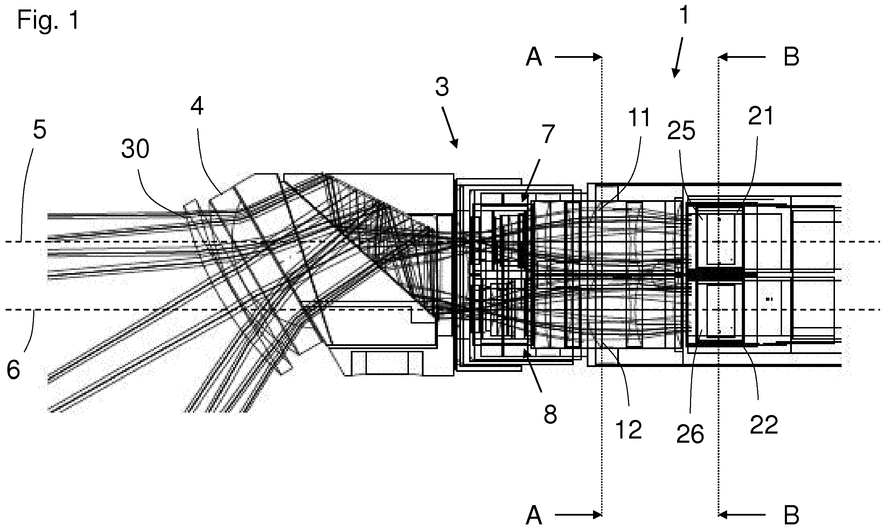

Fig. 1 zeigt schematisch einen Längsschnitt durch ein optisches System 3 eines seitwärts blickenden Stereo-Videoendoskops 1. Das optische System 3 umfasst einen ersten Linsensystemkanal 7 mit einer ersten optischen Achse 5 und einen zweiten Linsensystemkanal 8 mit einer zweiten optischen Achse 6. Die optischen Achsen 5, 6 sind parallel zueinander angeordnet. In dem ersten Linsensystemkanal 7 ist wenigstens ein erstes optisches Element 11 und in dem zweiten Linsensystemkanal 8 wenigstens ein zweites optisches Element 12 neben dem ersten optischen Element 11 angeordnet.

Beide Linsensystemkanäle 7, 8 umfassen mehrere optische Elemente, die aufgrund einer besseren Übersichtlichkeit in Fig. 1 nicht alle mit Bezugszeichen versehen sind. Das optische System umfasst zudem für jeden Linsensystemkanal 7, 8 jeweils ein Umlenkelement 25, 26 und einen Bildsensor 21, 22.

Lichtbündel, dargestellt durch jeweils drei Linien, fallen durch ein Eintrittsfenster 30 und eine Eintrittslinse 4 in das optische System 3 ein und werden anschließend in dem ersten Linsensystemkanal 7 und dem zweiten Linsensystemkanal 8 in Richtung der Umlenkelemente 25, 26 geführt. Das erste Umlenkelement 25 lenkt die Lichtbündel im ersten Linsensystemkanal 7 in Richtung aus der Zeichnungsebene heraus um, während das zweite Umlenkelement 26 die Lichtbündel im zweiten Linsensystemkanal 8 in Richtung in die Zeichnungsebene hinein lenkt. Auf diese Weise werden die Lichtbündel auf die parallel zur Zeichnungsebene angeordneten Bildsensoren 21, 22 abgebildet, welche das Licht der einfallenden Lichtbündel in Bildinformationen umwandeln.

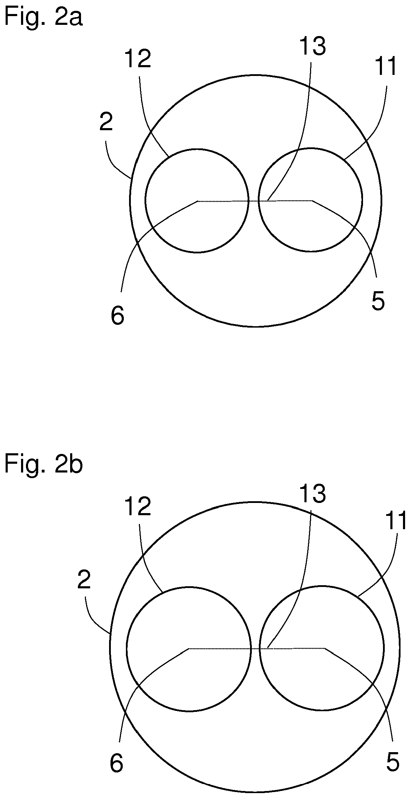

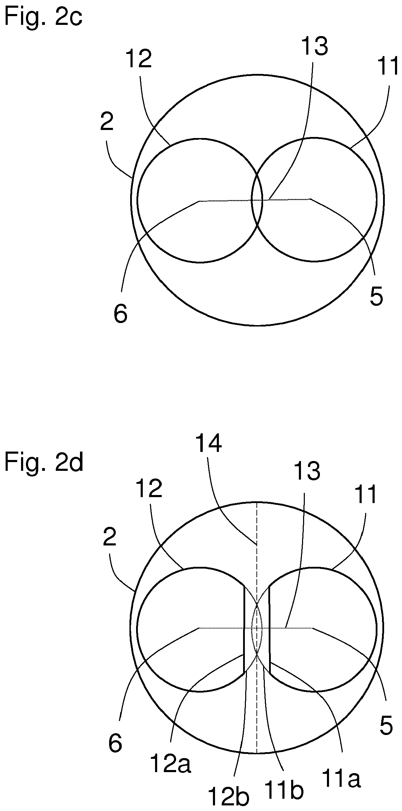

Die Fig. 2a - 2d zeigen jeweils schematische Querschnitte durch Stereo-Videoendoskope auf Höhe der optischen Elemente 11, 12. Ein schematischer Querschnitt durch das Stereo-Videoendoskop aus Fig. 1 entlang der Linie A - A ist in Fig. 2d gezeigt.

Fig. 2a zeigt einen Querschnitt durch das optische System 3 eines Stereo-Videoendoskops 1 nach dem Stand der Technik. Bei den optischen Elementen 11, 12 handelt es sich um Linsen, die einen kreisförmigen Querschnitt aufweisen. Die Zentren der Linsen, also die optischen Achsen 5, 6 der optischen Elemente 11, 12 fallen mit den optischen Achsen 5, 6 der Linsensystemkanäle 7, 8 zusammen. Der Abstand zwischen der ersten optischen Achse 5 und der zweiten optischen Achse 6, also die Länge der Verbindungslinie 13, ist entscheidend für die Stärke des stereoskopischen Effekts des Stereo-Videoendoskops 1. Ist der Abstand zu klein, so ist der von einem Betrachter wahrgenommene stereoskopische Effekt schwach. Ist der Abstand hingegen zu groß, so nimmt der Betrachter anstelle eines stereoskopischen Bilds zwei getrennte Bilder wahr. Um ein gutes stereoskopisches Bild zu erhalten, muss also ein geeigneter Abstand zwischen den optischen Achsen 5, 6 gewählt werden. Ein solcher Abstand liegt beispielsweise bei Stereo-Videoendoskopen mit einem Außendurchmesser von 10 mm bei etwa 2,5 bis 3 mm. Damit die optischen Elemente 11, 12 in dem Stereo-Videoendoskop 1 untergebracht werden können, muss der Durchmesser der optischen Elemente 11, 12 zudem so gewählt werden, dass diese in das Hüllrohr 2 passen.

Um diesen beiden Bedingungen gerecht zu werden, werden in optischen Systemen 3 nach dem Stand der Technik für gewöhnlich optische Elemente 11, 12 mit einem vergleichsweise kleinen Durchmesser gewählt. Um eine höhere Bildqualität zu erhalten, ist es jedoch notwendig, optische Elemente 11, 12 mit einem größeren Durchmesser zu verwenden, wie dies in Fig. 2b gezeigt ist. Um die optischen Elemente 11, 12 in dem Hüllrohr 2 unterzubringen, muss der Durchmesser des Hüllrohrs 2 vergrößert werden. Dies resultiert in einer Vergrößerung des Durchmessers des Stereo-Videoendoskops 1, was unerwünscht ist. Zudem ist in Fig. 2b der Abstand der optischen Achsen 5, 6 vergrößert, damit die optischen Elemente 11, 12 nebeneinander angeordnet werden können. Durch den größeren Abstand wird von einem Betrachter jedoch im Extremfall kein stereoskopisches Bild mehr, sondern zwei getrennte Bilder wahrgenommen. Würde hingegen der Abstand zwischen den optischen Achsen 5, 6 bei einer Vergrößerung der optischen Elemente 11, 12 gleichgehalten, so würden sich die optischen Elemente 11, 12 überschneiden, wie dies in Fig. 2c gezeigt ist.

Um dieses Problem zu lösen, werden die optischen Elemente 11, 12 mit einem sogenannten D-Cut versehen, wie er in Fig. 2d gezeigt ist. Die Querschnittsflächen der optischen Elemente 11, 12 haben die Form von Kreissegmenten, wobei die Zentren der umschreibenden Umfangskreise 11b, 12b mit den optischen Achsen 5, 6 der Linsensystemkanäle zusammenfallen. Die Kreissegmente sind so groß gewählt, dass sie diese Zentren, also die optischen Achsen 5, 6 einschließen. Die Kreissegmente werden von Kreissehnen 11a, 12a begrenzt. Die optischen Elemente 11, 12 sind so ausgerichtet, dass die durch die Kreissehnen 11a, 12a definierten Kanten einander zugewandt sind. Die optischen Elemente 11, 12 sind spiegelsymmetrisch in Bezug auf eine Mittellinie 14, die senkrecht zu den optischen Achsen 5, 6 und der Verbindungslinie 13 steht und mittig zwischen den optischen Elementen 11, 12 verläuft.

Durch das Ausgestalten der optischen Elemente 11, 12 in Form eines Kreissegments können die optischen Achsen 5, 6 näher aneinander angeordnet werden, als dies bei optischen Elementen 11, 12 mit gleichem Durchmesser und vollständig kreisförmigen Querschnitt der Fall wäre, wie in Fig. 2b gezeigt. Auf diese Weise wird auch mit großen optischen Elementen 11, 12 ein stereoskopisches Bild erzeugt. Zudem kann auch der Durchmesser des Hüllrohrs 2 kleiner gehalten werden. Im Vergleich zu dem in Fig. 2a gezeigten Fall mit kleineren optischen Elementen 11, 12 kann mehr Licht in Richtung der Bildsensoren 21, 22 geleitet werden. Dies resultiert in einer höheren Bildqualität.

Die Erfindung ist nicht auf optische Elemente 11, 12 beschränkt, die einen D-Cut in Form einer Kreissehne aufweisen. Die Erfindung umfasst ebenfalls Ausführungsformen, bei denen die optischen Elemente auf andere Weise so geformt sind, dass die Umfangskreise 11b, 12b der optischen Elemente 11, 12 überlappen. So ist es beispielsweise vorstellbar, dass die Kanten der optischen Elemente 11, 12 in dem Bereich, in dem die Kreissehnen 11a, 12a in den kreisförmigen Abschnitt der Kanten der optischen Elemente 11, 12 übergehen, abgerundet sind. Auch ovale Formen sind erfindungsgemäß umfasst.

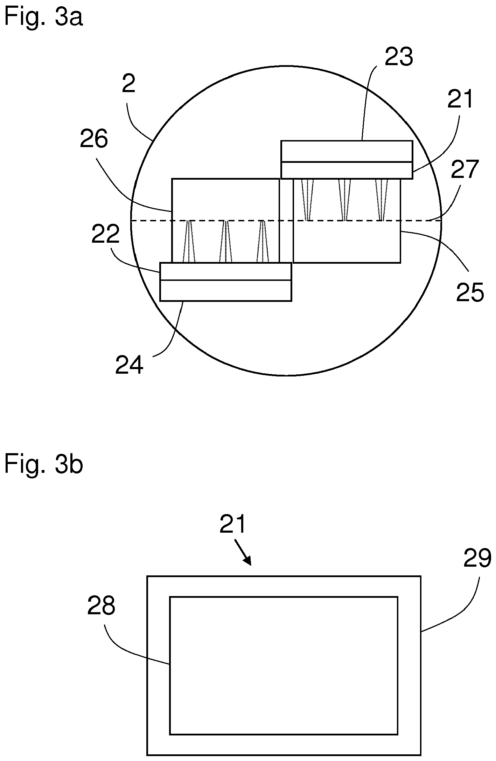

Fig. 3a zeigt einen Querschnitt durch das optische System 3 entlang der in Fig. 1 gezeigten Linie B - B mit den Bildsensoren 21, 22, bei denen es sich beispielsweise um CCD-Sensoren handelt. Aus technischen Gründen sind die Bildsensoren 21, 22 auf Trägern 23, 24 angeordnet. Beide Bildsensoren 21, 22 sind parallel zu einer Schnittfläche 27 ausgerichtet, wobei der erste Bildsensor 21 oberhalb und der zweite Bildsensor 22 unterhalb der Schnittfläche 27 angeordnet ist. Die Schnittfläche 27 wird aufgespannt durch die optischen Achsen 5, 6 und die Verbindungslinie 13, wie in den Fig. 2a - 2d gezeigt. Mittels der Umlenkelemente 25, 26, bei denen es sich beispielsweise um Prismen oder Spiegel handelt, werden einfallende Lichtbündel, angedeutet durch die Punktlinien, in Richtung der Bildsensoren 21, 22 umgelenkt.

Durch diese Anordnung der Bildsensoren 21, 22 können die Flächen der Bildsensoren 21, 22 so groß gewählt werden, dass diese sich in einer Projektion auf die Schnittfläche 27 überlappen. Dies ist entscheidend, da die Bildsensoren 21, 22 aus technischen Gründen eine lichtempfindliche Fläche 28 und einen lichtunempfindlichen Rand 29 aufweisen, wie dies am Beispiel des ersten Bildsensors 21 in Fig. 3b gezeigt ist. Bei großen Bildsensoren 21, 22 führt dies dazu, dass die Bildsensoren 21, 22 nicht mehr so nahe nebeneinander angeordnet werden können, dass ein für eine stereoskopische Wahrnehmung erforderlicher Abstand der optischen Achsen 5, 6 realisiert werden kann. Durch die in Fig. 3a gezeigte Anordnung wird dieses Problem gelöst, da sich die Ränder 29 und sogar die lichtempfindlichen Flächen 28 in einer Projektion auf die Schnittfläche 27 überschneiden können. Der Abstand der optischen Achsen 5, 6 kann also unabhängig von der Größe der Bildsensoren 21, 22 gewählt werden. Zudem ist es möglich, größere Bildsensoren 21, 22 zu verwenden und somit eine höhere Bildqualität zu erreichen.

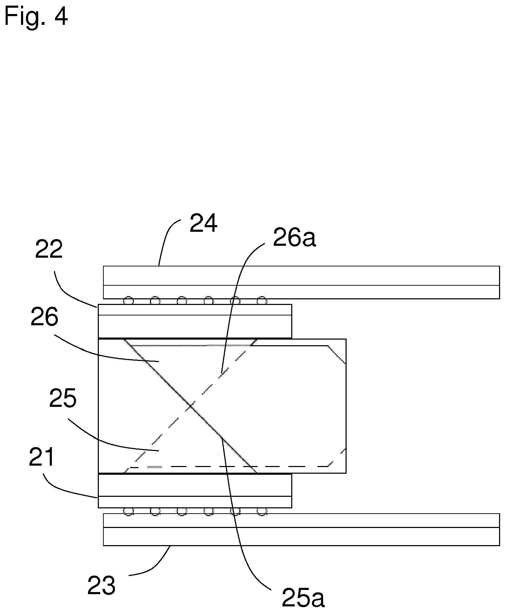

Fig. 4 zeigt schematisch eine Draufsicht auf die in Fig. 3a gezeigte Anordnung der Bildsensoren 21, 22 und der Umlenkelemente 25, 26. Die Ansicht entspricht somit einer Sicht aus der in Fig. 1 oben gelegenen Richtung. In dieser Draufsicht sind die Reflektionsflächen 25a, 26a der Umlenkelemente 25, 26 zu erkennen, an denen das einfallende Licht reflektiert und in Richtung des ersten Bildsensors 21 bzw. des zweiten Bildsensors 22 umgelenkt wird.

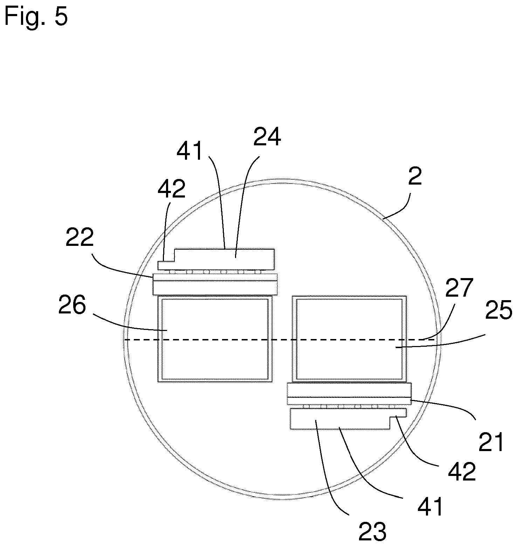

In Fig. 5 ist ein Querschnitt durch das optische System 3 gezeigt, der im Wesentlichen dem in Fig. 3a gezeigten Querschnitt entspricht, wobei jedoch eine Spiegelung um die Schnittfläche 27 vorliegt. Im Gegensatz zu Fig. 3a weisen die Träger 23, 24 in Fig. 5 jeweils einen in Bezug auf das Hüllrohr 2 nahen Bereich 42 und einen entfernten Bereich 41 auf. Die Dicke der Träger 23, 24 ist im nahen Bereich 42 im Vergleich zum entfernten Bereich 41 reduziert. Durch die dünnere Gestaltung des nahen Bereichs 42 werden die Träger 23, 24 raumsparender gestaltet. Dies ermöglicht es, beispielsweise den Durchmesser des Hüllrohrs 2 zu reduzieren oder, wie in Fig. 5 gezeigt, die Bildsensoren 21, 22 und die zugehörigen Träger 23, 24 zu vergrößern und so eine höhere Bildqualität zu erreichen.

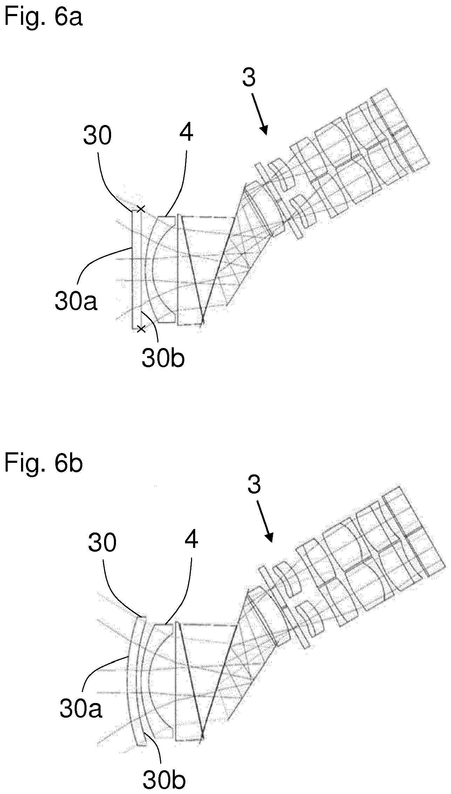

In Fig. 6a ist ein optisches System 3 eines Stereo-Videoendoskops 1 nach dem Stand der Technik schematisch dargestellt. Lichtbündel fallen durch eine plane Eintrittsfläche 30a und eine plane Austrittsfläche 30b des Eintrittsfenster 30 in das optische System 3 ein. Die Größe des Eintrittsfensters 30 bestimmt, wieviel Licht in das optische System 3 einfällt. Der Durchmesser des Eintrittsfensters 30 wird aber durch den Durchmesser des Stereo-Videoendoskops 1 begrenzt. Somit ist auch das Blickfeld des Endoskops 1 begrenzt. In Fig. 6a ist dies dadurch dargestellt, dass die äußersten dargestellten Lichtbündel, markiert mit einem "x", nicht in das optische System 3 einfallen können. Zur besseren Vergleichbarkeit mit der nachfolgenden Fig. 6b ist der hypothetische Strahlengang dieser Lichtbündel im optischen System 3 dennoch in Fig. 6a gezeigt.

Damit mehr Licht in das optische System 3 einfällt und der darstellbare Bildbereich vergrößert wird, wird gemäß der in Fig. 6b gezeigten Ausführungsform der Erfindung ein Eintrittsfenster 30 verwendet, dessen Eintrittsfläche 30a und Austrittsfläche 30b endliche und zueinander identische Krümmungsradien aufweisen. Dadurch wird bei gleichbleibendem Durchmesser die Oberfläche der Eintrittsfläche 30a vergrößert. Durch die Ausgestaltung der Eintrittsfläche 30a als gekrümmte Fläche können Lichtbündel aus einem größeren Blickfeld in das optische System 3 einfallen, wie im Vergleich von Fig. 6a mit Fig. 6b deutlich wird. Auf diese Weise wird eine Verbesserung der optischen Eigenschaften des optischen Systems 3 erreicht. Indem die Austrittsfläche 30b eine identische Krümmung zur Eintrittsfläche 30a aufweist, wird vermieden, dass das Eintrittsfenster 30 wie eine Linse wirkt. Ein negativer Einfluss des Eintrittsfensters 30 auf die optischen Eigenschaften des optischen Systems 3 durch einen unerwünschten Linseneffekt wird somit vermieden.

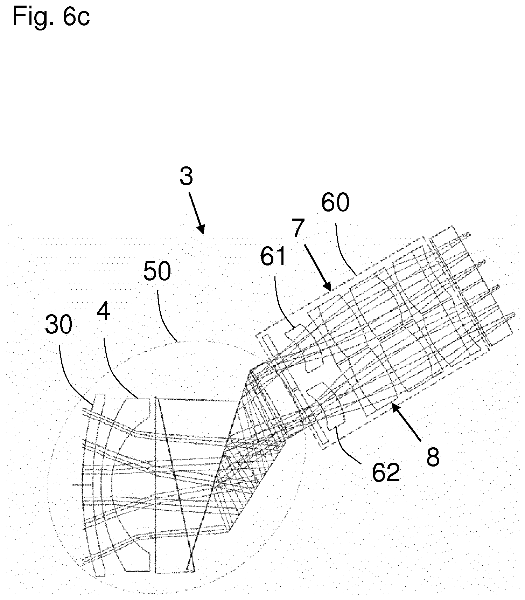

Fig. 6c zeigt eine weitere Ausführungsform eines optischen Systems 3. Für eine bessere Übersichtlichkeit ist die distale optische Baugruppe 50, durch die Licht in das optische System eindringt, und die proximale optische Baugruppe 60, die die Linsensystemkanäle 7, 8 umfasst, jeweils mit gestrichelten Linien kenntlich gemacht. In dieser Ausführungsform sind ein optisches Element 61 im ersten Linsensystemkanal 7 und ein, gegebenenfalls gleichartiges, optisches Element 62 im zweiten Linsensystemkanal 8 versetzt zueinander angeordnet. Auf diese Weise können unterschiedliche Strahlengänge des Lichts in der distalen optischen Baugruppe 50 ausgeglichen werden, so dass die Bildqualität der beiden Bilder angeglichen wird und ein stereoskopisches Bild mit höherer Bildqualität entsteht.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 1: Stereo-Videoendoskop
- 2: Hüllrohr
- 3: optisches System
- 4: Eintrittslinse
- 5: erste optische Achse
- 6: zweite optische Achse
- 7: erster Linsensystemkanal
- 8: zweiter Linsensystemkanal
- 11: erstes optisches Element
- 11a: erste Kreissehne
- 11b: erster Umfangskreis
- 12: zweites optisches Element
- 12a: zweite Kreissehne
- 12b: zweiter Umfangskreis
- 13: Verbindungslinie
- 14: Mittellinie
- 21: erster Bildsensor
- 22: zweiter Bildsensor
- 23: erster Träger
- 24: zweiter Träger
- 25: erstes Umlenkelement
- 25a: erste Reflektionsfläche
- 26: zweites Umlenkelement
- 26a: zweite Reflektionsfläche
- 27: Schnittfläche
- 28: lichtempfindliche Fläche
- 29: lichtunempfindlicher Rand
- 30: Eintrittsfenster
- 30a: Eintrittsfläche
- 30b: Austrittsfläche
- 41: entfernter Bereich
- 42: naher Bereich
- 50: proximale optische Baugruppe
- 60: distale optische Baugruppe
- 61: optisches Element
- 62: optisches Element

## Patentansprüche

1. Optisches System (3) für ein Stereo-Videoendoskop (1) mit einem ersten Linsensystemkanal (7) und einem zweiten Linsensystemkanal (8) für eine stereoskopische Darstellung eines außerhalb des Stereo-Videoendoskops (1) liegenden Bereichs, wobei die Linsensystemkanäle (7, 8) parallel zueinander angeordnet sind und jeweils ein oder mehrere optische Elemente (11, 12) in jeweils gleicher optischer Konfiguration umfassen, die jeweils in gleicher Position entlang einer ersten optischen Achse (5) des ersten Linsensystemkanals bzw. einer zweiten optischen Achse (6) des zweiten Linsensystemkanals nebeneinander angeordnet sind, wobei der erste Linsensystemkanal (7) wenigstens ein erstes optisches Element (11) und der zweite Linsensystemkanal (8) wenigstens ein dem ersten optischen Element (11) benachbartes zweites optisches Element (12) umfasst, wobei eine erste optische Achse (5) des ersten optischen Elements (11) mit der ersten optischen Achse (5) des ersten Linsensystemkanals (7) zusammenfällt und eine zweite optische Achse (6) des zweiten optischen Elements (12) mit der zweiten optischen Achse (6) des zweiten Linsensystemkanals (8) zusammenfällt, wobei die Querschnittsfläche des ersten optischen Elements (11) in einen ersten Umfangskreis (11b) und die Querschnittsfläche des zweiten optischen Elements (12) in einen zweiten Umfangskreis (12b) eingeschrieben sind, deren Zentren jeweils mit der ersten optischen Achse (5) des ersten optischen Elements (11) bzw. der zweiten optischen Achse (6) des zweiten optischen Elements (12) zusammenfallen und die einen maximalen Radius des ersten optischen Elements (11) bzw. des zweiten optischen Elements (12) bestimmen, wobei der erste Umfangskreis (11b) und der zweite Umfangskreis (12b) einander überlappen, wobei Umfangsformen des ersten optischen Elements (11) und des zweiten optischen Elements (12) von den sie umschreibenden ersten und zweiten Umfangskreisen (11b, 12b) derart abweichen, dass sich das erste optische Element (11) und das zweite optische Element (12) nicht berühren, **dadurch gekennzeichnet, dass** der erste Linsensystemkanal (7) einen ersten Bildsensor (21) und ein erstes Umlenkelement (25) und der zweite Linsensystemkanal (8) einen zweiten Bildsensor (22) und ein zweites Umlenkelement (26) umfassen, wobei der erste Bildsensor (21) oberhalb und der zweite Bildsensor (22) unterhalb einer von der ersten optischen Achse (5) des ersten optischen Elements (11) und der zweiten optischen Achse (6) des zweiten optischen Elements (12) aufgespannte Schnittfläche (27) angeordnet und beide Bildsensoren (21, 22) planparallel zur Schnittfläche (27) ausgerichtet sind, wobei das erste Umlenkelement (25) in den ersten Linsensystemkanal (7) einfallende Lichtbündel in Richtung des ersten Bildsensors (21) und das zweite Umlenkelement (26) in den zweiten Linsensystemkanal (8) einfallende Lichtbündel in Richtung des zweiten Bildsensors (22) ablenkt.

2. Optisches System (3) nach Anspruch 1, **dadurch gekennzeichnet, dass** die Querschnittsfläche des ersten optischen Elements (11) und die Querschnittsfläche des zweiten optischen Elements (12) jeweils die Form eines Kreissegments aufweisen, wobei die Querschnittsfläche des ersten optischen Elements (11) das Zentrum des ersten Umfangskreises (11b) und die Querschnittsfläche des zweiten optischen Elements (12) das Zentrum des zweiten Umfangskreises (12b) umfassen, wobei eine die Querschnittsfläche des ersten optischen Elements (11) begrenzende erste Kreissehne (11a) und eine die Querschnittsfläche des zweiten optischen Elements (12) begrenzende zweite Kreissehne (12a) jeweils senkrecht zu einer Verbindungslinie (13) zwischen den Zentren der Umfangskreise (11b, 12b) angeordnet sind.

3. Optisches System (3) nach Anspruch 1 oder 2, **dadurch gekennzeichnet, dass** das erste optische Element (11) zum zweiten optischen Element (12) spiegelsymmetrisch in Bezug auf eine Mittellinie (14) ist, die mittig zwischen den Zentren der Umfangskreise (11b, 12b) angeordnet ist und senkrecht auf einer Verbindungslinie (13) zwischen den Zentren der Umfangskreise (11b, 12b) steht.

4. Optisches System (3) nach einem der Ansprüche 1 bis 3, **dadurch gekennzeichnet, dass** die die Querschnittsflächen umschreibenden Umfangskreise (11b, 12b) der optischen Elemente (11, 12) einen Durchmesser von wenigstens 3,4 mm aufweisen und ein Abstand zwischen den Zentren der Umfangskreise (11b, 12b) 2,5 mm bis 3 mm beträgt.

5. Optisches System (3) nach einem der Ansprüche 1 bis 4, **dadurch gekennzeichnet, dass** die Bildsensoren (21, 22) jeweils eine lichtempfindliche Fläche (28) und einen lichtunempfindlichen Rand (29) umfassen, wobei sich die Bildsensoren (21, 22), insbesondere die lichtunempfindlichen Ränder (29) der Bildsensoren (21, 22), in einer Projektion auf die Schnittfläche (27) überlappen.

6. Optisches System (3) nach einem der Ansprüche 1 bis 5, **dadurch gekennzeichnet, dass** die Bildsensoren (21, 22) jeweils auf einem Träger (23, 24) angeordnet sind, wobei die Träger (23, 24) in einem in Bezug auf ein Hüllrohr (2) des optischen Systems (3) nahen Bereich (42) dünner als in einem in Bezug auf das Hüllrohr (2) entfernten Bereich (41) sind.

7. Optisches System (3) nach einem der Ansprüche 1 bis 6, **dadurch gekennzeichnet, dass** Licht durch ein Eintrittsfenster (30) in das optische System (3) einfällt, wobei das Eintrittsfenster (30) eine Eintrittsfläche (30a) und eine Austrittsfläche (30b) umfasst und die Eintrittsfläche (30a) und die Austrittsfläche (30b) endliche und zueinander identische Krümmungsradien aufweisen.

8. Stereo-Videoendoskop (1) umfassend ein optisches System (3) nach einem der Ansprüche 1 bis 7.

## Claims

1. An optical system (3) for a stereo video endoscope (1), comprising a first lens system channel (7) and a second lens system channel (8) for a stereoscopic representation of a region lying outside of the stereo video endoscope (1), wherein the lens system channels (7, 8) are arranged in parallel to one another and each comprise one or more optical elements (11, 12) in a respective identical optical configuration, which are each arranged next to one another in the same position along a first optical axis (5) of the first lens system channel or a second optical axis (6) of the second lens system channel, wherein the first lens system channel (7) comprises at least one first optical element (11) and the second lens system channel (8) comprises at least one second optical element (12) neighbouring the first optical element (11), wherein a first optical axis (5) of the first optical element (11) coincides with the first optical axis (5) of the first lens system channel (7) and a second optical axis (6) of the second optical element (12) coincides with the second optical axis (6) of the second lens system channel (8), wherein the cross-sectional area of the first optical element (11) is inscribed in a first circumferential circle (11b) and the cross-sectional area of the second optical element (12) is inscribed in a second circumferential circle (12b), the centres of which each coincide with the first optical axis (5) of the first optical element (11) and, respectively, the second optical axis (6) of the second optical element (12), and which determine a maximum radius of the first optical element (11) and, respectively, the second optical element (12), wherein the first circumferential circle (11b) and the second circumferential circle (12b) overlap one another, wherein circumferential shapes of the first optical element (11) and the second optical element (12) deviate from the first and second circumferential circles (11b, 12b) circumscribing them in such a way that the first optical element (11) and the second optical element (12) do not touch, **characterised in that** the first lens system channel (7) comprises a first image sensor (21) and a first deflection element (25), and the second lens system channel (8) comprises a second image sensor (22) and a second deflection element (26), wherein the first image sensor (21) is arranged above and the second image sensor (22) is arranged below a sectional plane (27) spanned by the first optical axis (5) of the first optical element (11) and the second optical axis (6) of the second optical element (12) and both image sensors (21, 22) are aligned plane parallel to the sectional plane (27), wherein the first deflection element (25) diverts light beams incident in the first lens system channel (7) in the direction of the first image sensor (21) and the second deflection element (26) diverts light beams incident in the second lens system channel (8) in the direction of the second image sensor (22).

2. The optical system (3) according to Claim 1, **characterised in that** the cross-sectional area of the first optical element (11) and the cross-sectional area of the second optical element (12) each have the form of a circular segment, wherein the cross-sectional area of the first optical element (11) comprises the centre of the first circumferential circle (11b) and the cross-sectional area of the second optical element (12) comprises the centre of the second circumferential circle (12b), wherein a first chord (11a) delimiting the cross-sectional area of the first optical element (11) and a second chord (12a) delimiting the cross-sectional area of the second optical element (12) are each arranged perpendicular to a connecting line (13) between the centres of the circumferential circles (11b, 12b).

3. The optical system (3) according to Claim 1 or 2, **characterised in that** the first optical element (11) is mirror-symmetrical to the second optical element (12) with respect to a centre line (14) which is arranged centrally between the centres of the circumferential circles (11b, 12b), and is perpendicular to a connecting line (13) between the centres of the circumferential circles (11b, 12b).

4. The optical system (3) according to any one of Claims 1 to 3, **characterised in that** the circumferential circles (11b, 12b) of the optical elements (11, 12) circumscribing the cross-sectional areas have a diameter of at least 3.4 mm, and a distance between the centres of the circumferential circles (11b, 12b) is 2.5 mm to 3 mm.

5. The optical system (3) according to any one of Claims 1 to 4, **characterised in that** the image sensors (21, 22) each comprise a light-sensitive surface (28) and a light-insensitive edge (29), wherein the image sensors (21, 22), in particular the light-insensitive edges (29) of the image sensors (21, 22), overlap in a projection onto the sectional plane (27).

6. The optical system (3) according to any one of Claims 1 to 5, **characterised in that** the image sensors (21, 22) are each arranged on a support (23, 24), wherein the supports (23, 24) are thinner in a near region (42) with respect to a casing tube (2) of the optical system (3) than in a distant region (41) with respect to the casing tube (2).

7. The optical system (3) according to any one of Claims 1 to 6, **characterised in that** light enters the optical system (3) through an inlet window (30), wherein the inlet window (30) comprises an inlet surface (30a) and an outlet surface (30b) and the inlet surface (30a) and the outlet surface (30b) have finite radii of curvature which are identical to one another.

8. A stereo video endoscope (1) comprising an optical system (3) according to any one of Claims 1 to 7.

## Revendications

1. Système optique (3) pour un vidéo-endoscope stéréo (1) ayant un premier canal (7) à système de lentilles et un deuxième canal (8) à système de lentilles pour une représentation stéréoscopique d'une zone située à l'extérieur du vidéo-endoscope stéréo (1), les canaux (7, 8) à système de lentilles étant agencés parallèlement l'un à l'autre et comprenant chacun un ou plusieurs éléments optiques (11, 12) dans une configuration optique respectivement identique, qui sont agencés respectivement dans la même position, l'un à côté de l'autre, le long d'un premier axe optique (5) du premier canal à système de lentilles ou d'un deuxième axe optique (6) du deuxième canal à système de lentilles, le premier canal (7) à système de lentilles comprenant au moins un premier élément optique (11) et le deuxième canal (8) à système de lentilles comprenant au moins un deuxième élément optique (12) voisin du premier élément optique (11), un premier axe optique (5) du premier élément optique (11) coïncidant avec le premier axe optique (5) du premier canal (7) à système de lentilles et un deuxième axe optique (6) du deuxième élément optique (12) coïncidant avec le deuxième axe optique (6) du deuxième canal (8) à système de lentilles, la surface en section transversale du premier élément optique (11) étant inscrite dans un premier cercle périphérique (11b) et la surface en section transversale du deuxième élément optique (12) étant inscrite dans un deuxième cercle périphérique (12b), dont les centres sont respectivement alignés avec le premier axe optique (5) du premier élément optique (11) et le deuxième axe optique (12b) du deuxième axe optique (6) du deuxième élément optique (12) et qui définissent un rayon maximal du premier élément optique (11) ou du deuxième élément optique (12), le premier cercle périphérique (11b) et le deuxième cercle périphérique (12b) se chevauchant l'un l'autre, les formes périphériques du premier élément optique (11) et du deuxième élément optique (12) s'écartant des premier et deuxième cercles périphériques (11b, 12b) qui les circonscrivent de telle sorte que le premier élément optique (11) et le deuxième élément optique (12) ne se touchent pas, **caractérisé en ce que** le premier canal (7) à système de lentilles comprend un premier capteur d'image (21) et un premier élément de déviation (25) et le deuxième canal (8) à système de lentilles comprend un deuxième capteur d'image (22) et un deuxième élément de déviation (26), le premier capteur d'image (21) étant disposé au-dessus, et le deuxième capteur d'image (22) au-dessous, d'une surface d'intersection (27) définie par le premier axe optique (5) du premier élément optique (11) et le deuxième axe optique (6) du deuxième élément optique (12), et les deux capteurs d'image (21, 22) étant orientés dans des plans parallèles à la surface d'intersection (27), le premier élément de déviation (25) déviant les faisceaux lumineux incidents dans le premier canal (7) à système de lentilles en direction du premier capteur d'images (21) et le deuxième élément de déviation (26) déviant les faisceaux lumineux incidents dans le deuxième canal (8) à système de lentilles en direction du deuxième capteur d'images (22).

2. Système optique (3) selon la revendication 1, **caractérisé en ce que** la surface en coupe transversale du premier élément optique (11) et la surface en coupe transversale du deuxième élément optique (12) présentent chacune la forme d'un segment de cercle, la surface en coupe transversale du premier élément optique (11) comprenant le centre du premier cercle périphérique (11b) et la surface en coupe transversale du deuxième élément optique (12) comprenant le centre du deuxième cercle périphérique (12b), une première corde de cercle (11a) qui délimite la surface de section transversale du premier élément optique (11) et une deuxième corde de cercle (12a) qui délimite la surface de section transversale du deuxième élément optique (12) étant respectivement disposées perpendiculairement à une ligne (13) de jonction des centres des cercles périphériques (11b, 12b).

3. Système optique (3) selon la revendication 1 ou la revendication 2, **caractérisé en ce que** le premier élément optique (11) est symétrique au deuxième élément optique (12) par rapport à une ligne centrale (14) située à mi-chemin entre les centres des cercles périphériques (11b, 12b) et perpendiculaire à une ligne (13) de jonction des centres des cercles périphériques (11b, 12b).

4. Système optique (3) selon l'une des revendications 1 à 3, **caractérisé en ce que** les cercles périphériques (11b, 12b) des éléments optiques (11, 12) circonscrivant les surfaces en section transversale ont un diamètre d'au moins 3,4 mm et une distance entre les centres des cercles périphériques (11b, 12b) allant de 2,5 mm à 3 mm.

5. Système optique (3) selon l'une des revendications 1 à 4, **caractérisé en ce que** les capteurs d'image (21, 22) comprennent chacun une surface photosensible (28) et un bord non photosensible (29), les capteurs d'image (21, 22), en particulier les bords non photosensibles (29) des capteurs d'image (21, 22), se chevauchant dans une projection sur la surface d'intersection (27).

6. Système optique (3) selon l'une des revendications 1 à 5, **caractérisé en ce que** les capteurs d'images (21, 22) sont agencés chacun sur un support (23, 24), les supports (23, 24) étant plus fins dans une zone (42) proche par rapport à un tube enveloppe (2) du système optique (3) que dans une zone (41) éloignée par rapport au tube enveloppe (2).

7. Système optique (3) selon l'une des revendications 1 à 6, **caractérisé en ce que** la lumière est incidente dans le système optique (3) à travers une fenêtre d'entrée (30), la fenêtre d'entrée (30) comprenant une face d'entrée (30a) et une face de sortie (30b), la face d'entrée (30a) et la face de sortie (30b) présentant des rayons de courbure finis et identiques entre eux.

8. Vidéo-endoscope stéréoscopique (1) comprenant un système optique (3) selon l'une quelconque des revendications 1 à 7.
